# EUROPÄISCHE PATENTANMELDUNG

(11) **EP 0 841 043 A2**
(43) Veröffentlichungstag der Anmeldung: **13.05.1998**
(21) Anmeldenummer: 97119134.1
(22) Anmeldetag: 03.11.1997
(51) Int. Cl.: A61F 2/76

(54) **Orthopädische Klemmverbindung**

(30) Priorität: 06.11.1996 DE 19645679
(71) Anmelder: Otto Bock Orthopädische Industrie Besitz- und Verwaltungs-Kommanditgesellschaft, 37115 Duderstadt (DE)
(72) Erfinder: Anhalt, Klaus-Peter, 37434 Rhumspringe (DE); Deinert, Jürgen, 37115 Duderstadt (DE)
(74) Vertreter: Gramm, Werner, Prof. Dipl.-Ing.

(57) **Zusammenfassung**

Die Erfindung betrifft eine orthopädische Klemmverbindung als Funktionselement zur Kraftübertragung in einer Prothese, bestehend aus einer Rohrmuffe und einem Leichtmetallrohr. Zur Erhöhung der dynamischen Belastbarkeit wird erfindungsgemäß vorgeschlagen, daß die Kontaktfläche zwischen Rohrmuffe und Leichtmetallrohr mit einer Zwischenschicht versehen ist, die aus einem niedermolekularen Trägermaterial aufgebaut ist, in das Schmierstoffe und/oder Hilfsstoffe eingebettet sind.

## Beschreibung

Die Erfindung betrifft eine orthopädische Klemmverbindung als Funktionselement zur Kraftübertragung in einer Prothese, bestehend aus einer Rohrmuffe und einem Leichtmetallrohr.

Eine derartige Klemmverbindung läßt sich der EP 0 694 295 A2 entnehmen. Offenbart ist hier eine axial geschlitzte, über eine Spannschraube umfangsverspannbare Rohrmuffe, an deren eines Ende sich ein ringförmiger Kupplungsabschnitt anschließt, der auf seinem Umfang vier Gewindebohrungen zur Aufnahme je einer Kupplungs- und Justierschraube und stirnseitig eine ringförmige Kugelpfanne aufweist zur Anlage an einer Kugelkalotte eines Justierkernes, der in Kupplungsstellung mit einem als mehrflächige Pyramide ausgebildeten Ansatz bis in den Klemmbereich der Klemmschrauben ragt. Der dem Kupplungsabschnitt gegenüberliegende freie Endabschnitt der Rohrmuffe ist auf seiner inneren Ringfläche mit einer Kunststoff-Klemmanschette bestückt, die als separater Ring oder aber als Innenbeschichtung ausgebildet sein kann. Die axiale Länge der Kunststoff-Klemmanschette entspricht etwa einem Fünftel der Axiallänge der Rohrmuffe. Durch diese Ausführungsform wurde erreicht, daß die Klemmung nicht mehr unmittelbar am Rohrende sondern in einem Abstand hiervon erfolgte, so daß der zwischen der Kunststoff-Klemmanschette und dem Kupplungsabschnitt liegende Rohrabschnitt elastischen Verformungen unterworfen werden kann. Im Bereich der höchsten Relativbewegung zwischen Rohrmuffe und Rohrende wird ein metallischer Kontakt zwischen diesen beiden Teilen durch die Kunststoff-Klemmanschette unterbunden. Dynamische Biegeversuche haben ergeben, daß sich hierdurch die Lebensdauer der genannten Bauteile erheblich verlängert.

Diese vorbekannte Lösung hat sich grundsätzlich bewährt, jedoch müssen zur Erlangung der genannten Vorteile andere Nachteile in Kauf genommen werden. So verlangt die Manschette als separater Kunststoffring eine Verlängerung des Adapters und somit höhere Fertigungskosten und ein höheres Gewicht. Die Kunststoffmanschette selbst kann fertigungsbedingte Spannungen und Unrundheiten aufweisen, was zu Problemen bei der Montage führen kann. Bei der Ausführung der Manschette als Kunststoffschicht sind die erreichbaren Toleranzen ungenügend; es muß daher ein Übermaß aufgetragen und anschließend spanend nachgearbeitet werden.

Die nachfolgende Erfindung beruht ferner auf der Erkenntnis, daß eine Kunststoffmanschette Reibkorrosion nur bedingt unterdrücken kann.

Der Erfindung liegt somit die Aufgabe zugrunde, die eingangs beschriebene Klemmverbindung hinsichtlich einer höheren dynamischen Belastbarkeit zu verbessern und zugleich eine gute Lösbarkeit zwischen Adapter und Leichtmetallrohr auch noch nach langfristiger Anwendung sicherzustellen.

Diese Aufgabe wird erfindungsgemäß dadurch gelöst, daß die Kontaktfläche zwischen Rohrmuffe und Leichtmetallrohr mit einer Zwischenschicht versehen ist, die aus einem niedermolekularen Trägermaterial aufgebaut ist, in das Schmierstoffe und/oder Hilfsstoffe eingebettet sind.

Unter dem Begriff "niedermolekular" wird ein Molekulargewicht verstanden, das kleiner ist als bei polymeren Stoffen und somit kleiner 5000 g/Mol betragen soll.

Die Funktion des Trägermaterials liegt im Verbund zum Untergrund, in der mechanischen Festigkeit und der Verschleißbeständigkeit. Schmierstoffe verhindern metallischen Reibkontakt, schützen die Rohroberfläche vor mechanischer Schädigung, ermöglichen stick-slip-freie Mikrobewegungen, führen zur Reibwertverminderung und vermindern auch Reibkorrosion. Hierbei hat sich überraschenderweise herausgestellt, daß die dynamische Strukturfestigkeit der Klemmverbindung durch Zugabe von Schmierstoffen signifikant erhöht werden kann. Dabei reicht gewöhnlich die Oberflächenrauhigkeit einer Metallfläche aus, um ausreichend Schmierstoffe aufzunehmen.

Hilfsstoffe dienen der Verträglichkeit und Haftung zwischen Schmierstoff, Trägermaterial und/oder Adapter- oder Rohrmaterial.

Bei der alleinigen Verwendung eines Schmierstoffs ohne Zusatz eines haftverbessernden Binders oder Benetzungsmittels sollte ein an Metallflächen selbständig haftendes Schmiermittel, wie z. B. MoS₂ eingesetzt werden. Die so erzielbaren Schichtstärken sind naturgemäß nur sehr gering (ca. 1 µm); es konnte aber trotzdem eine Verbesserung der dynamischen Strukturfestigkeit festgestellt werden. Dies wird dadurch erklärt, daß aus den Poren der Metalloberflächen Schmierstoff in die Grenzfläche nachgeliefert werden kann.

Es hat sich zudem herausgestellt, daß die adsorbierte Schmierstoffmenge durch Zugabe von Benetzungsmitteln gesteigert und somit eine Erhöhung der Schmierdauer erreicht werden kann.

Eine verbesserte dynamische Strukturfestigkeit kann auch erreicht werden, indem der Schmierstoff ganz oder teilweise durch Antioxidantien ersetzt wird. Dieser Effekt ist nur dadurch zu erklären, daß mit Antioxidantien nicht nur die Reibkorrosion vermindert wird, sondern auch das Ruckgleiten bei Mikrobewegungen zwischen Leichtmetallrohr und Rohrmuffe unterdrückt werden kann.

Durch Zugabe eines Bindemittels kann die Schichtstärke erhöht werden, was eine bessere Trennung der Metallflächen bedeutet. Außerdem sind höhere Schichtstärken notwendig, wenn die Schicht zur Vermeidung von Kontaktkorrosion elektrisch isolierend wirken soll. Durch den Einsatz von Bindersystemen kann der Schmierstoff auch besser auf der Grenzfläche gehalten werden, wodurch sich die Wiederverwendbarkeit vereinfacht. Zudem können bei Verwendung von Bindersystemen auch niederviskose Schmierstoffe eingesetzt werden.

Bindersysteme erlauben den Aufbau von Schichtstärken bis 1 mm, wodurch Fertigungstoleranzen ausgeglichen und eine gleichmäßige Kraftübertragung erreicht werden kann. Bei hohen Schichtstärken sind allerdings neben den tribologischen Eigenschaften auch die mechanischen Eigenschaften des Systems Schmierstoff-Binder von Bedeutung, wobei vor allem die Zähigkeit und das Kaltflußverhalten die dynamische Strukturfestigkeit der Klemmverbindung beeinflussen.

Als Bindersystem können sehr unterschiedliche Stoffe eingesetzt werden; es müssen lediglich die Verträglichkeit mit den eingesetzten Schmierstoffen sowie die Haftung zum Metalluntergrund bei Ausführung als Beschichtung gewährleistet werden. Bewährt haben sich anorganische sowie organische Binder. Besonders vorteilhaft haben sich anorganische Binder und duromere Harze zur Erzeugung von Schichten bis 50 µm bewährt. Thermoplastische Binder, aber auch Binder auf Teerbasis eignen sich dagegen aufgrund der Zähigkeit und des Fließverhaltens für Schichtstärken bis 1 mm.

Über die Art und die Konzentration von Schmierstoff und Binder kann ein definierter Reibwert eingestellt werden. Dieser darf unter den Anwendungsbedingungen nicht unter µ = 0,3 absinken, da ansonsten nicht mehr ausreichend Kraft übertragen werden kann. Bei fluorhaltigen Bindersystemen (z. B. FEP, PCTFE) kann das Binder/Schmierstoffverhältnis sehr groß werden; z. B. beträgt das Verhältnis bei der Kombination FEP/Silikonöl 50:1.

Neben der Verwendung von Schmierstoffen und/oder Antioxidantien haben sich sogenannte Haftstoffe zum Einsatz in der Schicht zwischen Leichtmetallrohr und Rohrmuffe bewährt. Diese Stoffe verhindern Mikrobewegungen zwischen Rohr und Muffe bei der dynamischen Belastung der Prothese. Sie weisen daher einen den Schmierstoffen entgegengesetzten Wirkmechanismus auf und können in ihrer Funktionalität und ihrem Aufbau mit Werkstoffen für Bremselemente verglichen werden.

Besonders vorteilhaft hat sich aber die Erhöhung der Haftreibung durch das Herstellen von Formschluß erwiesen. Hierfür müssen Stoffe in der Zwischenschicht eingesetzt werden, die inkompressibel sind, eine höhere Härte aufweisen als das zu klemmende Leichtmetallrohr und gleichzeitig einen starken Verbund mit dem Kernmaterial der Rohrmuffe eingehen. Beim Klemmvorgang können dann die Rauhigkeitsspitzen der Zwischenschicht in die Oberfläche des Leichtmetallrohrs eingedrückt werden, wodurch der Formschluß hergestellt wird.

Allerdings erweisen sich Haftschichten, die über Formschluß wirken, als abrasiv, wenn die gesteigerte Haftreibung durch Beaufschlagung eines unzulässig hohen Drehmoments überwunden wird und es zu Mikrobewegungen in der Klemmverbindung kommt.

Daher hat es sich auch bei den Haftschichten als vorteilhaft herausgestellt, in die Poren Schmierstoffe einzulagern. Diese werden bei Bewegungen zwischen Leichtmetallrohr und Zwischenschicht aus den Poren geschoben und vermindern so eine abrasive Schädigung bei Überbelastung der Prothese. Eine derart aufgebaute Zwischenschicht weist praktisch einen erhöhten Haftreibungskoeffizienten bei gleichzeitig vermindertem Gleitreibungskoeffizienten auf.

Auch bei Haftstoffen kann es vorteilhaft sein, durch vollständige oder teilweise Substitution des Schmierstoffs mit Antioxidantien Korrosion zu unterdrücken.

Die Applikation der erfindungsgemäßen Schmierstoffschicht ist je nach System mit unterschiedlichen Methoden durchführbar. Niederviskose Systeme können gezielt mit Pinsel oder Schwamm aufgetragen werden. Auch sind Tauch-, Reib- oder Spritzverfahren denkbar. Gewöhnlich muß die Oberfläche nicht vorbehandelt werden; bei Ausführung als Beschichtung sollte aber entfettet werden. Die Schmierstoffschicht kann über automatisierte Fertigungsverfahren aber auch manuell aufgetragen werden.

Folgende Ausführungsformen haben sich als besonders vorteilhaft herausgestellt:

### Beispiel 1:

- Material Rohrmuffe:: Stahl
- Material Rohrelement:: Aluminium
- Schichtstärke:: ca. 1 µm
- Trägermaterial:: 50 % Stahl
- Gleitmittel:: 49 % MoS₂
- Benetzungsmittel:: 1 % Alkylbenzolsulfonat

### Beispiel 2:

- Material Rohrmuffe:: Stahl
- Material Rohrelement:: Aluminium
- Schichtstärke:: ca. 4 µm
- Trägermaterial:: 25 % Bariumsulfat
- Gleitmittel:: 70 % MoS₂
- Antioxidationsmittel:: 5 % NN'-Dinaphtyl-p-phenylenamin (Aromatisches Amin)

### Beispiel 3:

- Material Rohrmuffe:: Titan
- Material Rohrelement:: Aluminium
- Schichtstärke:: ca. 15 µm
- Trägermaterial:: 25 % Stahl
- Hilfsstoff:: 25 % Ungesättigte Polyesterharze
- Gleitmittel:: 45 % PTFE/MoS₂-Gemisch
- Antioxidationsmittel:: 5 % 2,6-Di-tert-butyl-4-nonylphenol

### Beispiel 4:

- Material Rohrmuffe:: Aluminium
- Material Rohrelement:: Aluminium
- Schichtstärke:: ca. 100 µm
- Trägermaterial:: 30 % Aluminiumoxid
- Hilfsstoff:: 40 % PVDF
- Gleitmittel:: 10 % PTFE 10 % Li-Seife
- Antioxidationsmittel:: 10 % Tris(2,4-di-t-butylphenyl)phosphit (Phosphit)

### Beispiel 5:

- Material Rohrmuffe:: Stahl
- Material Rohrelement:: Aluminium
- Schichtstärke:: ca. 1 µm
- Trägermaterial:: 50 % Stahl
- Hilfsstoff:: 49 % PE-Wachs
- Gleitmittel:: 1 % Alkylbenzolsulfonat

### Beispiel 6:

- Material Rohrmuffe:: Stahl
- Material Rohrelement:: Aluminium
- Schichtstärke:: ca. 250 µm
- Trägermaterial (Haftschicht):: 90 % Gew.-% Quarz/Korund
- Gleitmittel:: 10 % Gew.-% Graphit

### Beispiel 7:

- Material Rohrmuffe:: Stahl
- Material Rohrelement:: Aluminium
- Schichtstärke:: ca. 1 mm
- Manschette: Trägermaterial:: 75 Gew.-% Aluminiumoxid
- Gleitmittel:: 23 Gew.-% Molybdänsulfid 2 Gew.-% Silikonöl

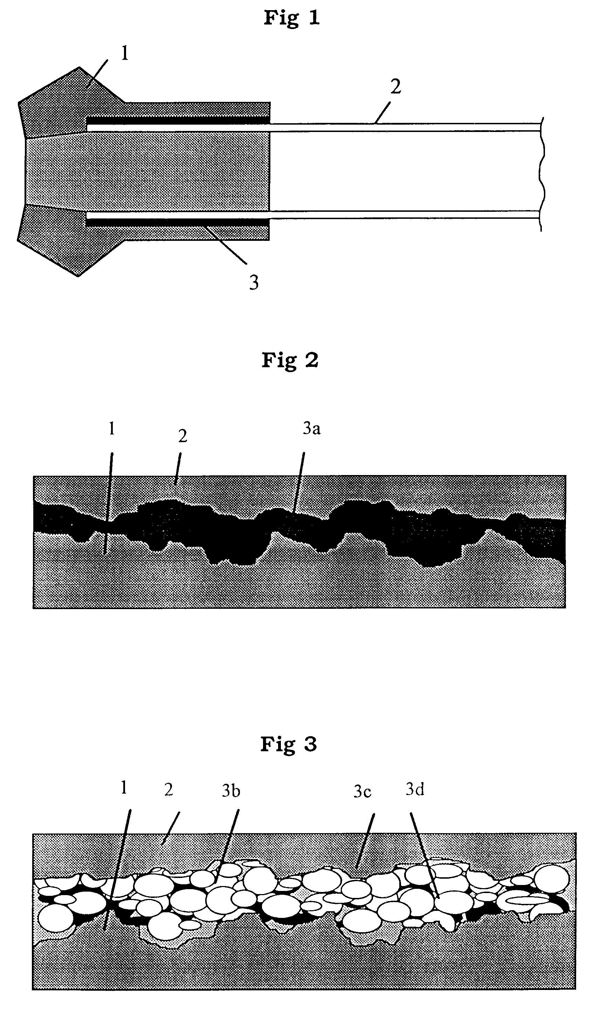

Figur 1 zeigt in schematischer Darstellung im Längsschnitt eine orthopädische Klemmverbindung als Funktionselement zur Kraftübertragung in einer Prothese, bestehend aus einer Rohrmuffe 1 aus Titan und aus einem Leichtmetallrohr 2 aus Aluminium. Zwischen diesen beiden Bauteilen ist eine Zwischenschicht 3 vorgesehen, die etwa 50 µm stark ist.

Figur 2 zeigt in stark vergrößertem Maßstab den in Figur 1 dargestellten Schnittbereich zwischen Rohrmuffe 1 und Leichtmetallrohr 2 mit der aus einem Trägermaterial mit eingebettetem MoS₂ als Schmierstoff 3a gebildeten Zwischenschicht.

Figur 3 zeigt in einer Darstellung gemäß Figur 2 die Grenzschicht zwischen der Rohrmuffe 1 und dem Leichtmetallrohr 2 mit einer zwischengeschalteten Schmierstoffschicht aus PTFE 3b und Phosphit 3c mit Polyesterharz als Hilfsstoff und Bariumsulfat 3d als Trägermaterial.

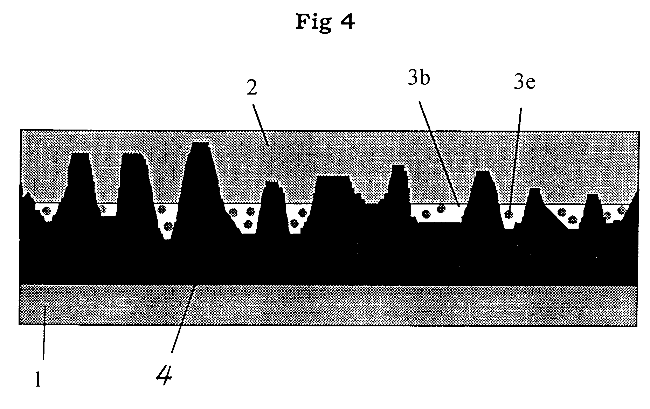

Figur 4 zeigt wiederum in stark vergrößerter Darstellung die Grenzschicht zwischen der Rohrmuffe 1 und dem Leichtmetallrohr 2 mit einem silikatischen Trägermaterial 4 sowie PTFE 3b als Schmierstoff und einem Alkylphenol 3e als Antioxidationsmittel.

## Patentansprüche

1. Orthopädische Klemmverbindung als Funktionselement zur Kraftübertragung in einer Prothese, bestehend aus einer Rohrmuffe (1) und einem Leichtmetallrohr (2), **dadurch gekennzeichnet**, daß die Kontaktfläche zwischen Rohrmuffe (1) und Leichtmetallrohr (2) mit einer Zwischenschicht (3, 4) versehen ist, die aus einem niedermolekularen Trägermaterial aufgebaut ist, in das Schmierstoffe und/oder Hilfsstoffe eingebettet sind.

2. Orthopädische Klemmverbindung nach Anspruch 1, **dadurch gekennzeichnet**, daß die Schmierstoffe Dicarbonsäureester, Fettsäuren, Fettsäureester, Fettsäureamide, Metallseifen, Silikonöle oder Molybdänsulfid bzw. Kombinationen dieser Stoffe enthalten.

3. Orthopädische Klemmverbindung nach Anspruch 1, **dadurch gekennzeichnet**, daß die Schmierstoffe Polyolefinwachse, Paraffine oder Fluorpolymere bzw. Kombinationen aus diesen Stoffen enthalten.

4. Orthopädische Klemmverbindung nach Anspruch 1, **dadurch gekennzeichnet**, daß das Trägermaterial anorganisch aufgebaut ist.

5. Orthopädische Klemmverbindung nach Anspruch 1 und 4, **dadurch gekennzeichnet**, daß das Trägermaterial aus Metallen, Metallsalzen, Metalloxiden oder aus keramischen Stoffen aufgebaut ist.

6. Orthopädische Klemmverbindung nach Anspruch 1 und 4, **dadurch gekennzeichnet**, daß das Trägermaterial aus Sulfaten, Aluminaten, Silikaten, Ferriten, Nickel und/oder Phosphor aufgebaut ist.

7. Orthopädische Klemmverbindung nach Anspruch 1, **dadurch gekennzeichnet**, daß die Zwischenschicht (3, 4) Antioxidantien enthält.

8. Orthopädische Klemmverbindung nach Anspruch 7, **dadurch gekennzeichnet**, daß die Antioxidantien, Alkylphenole, Hydroxyphenylpropionate, Hydroxybenzyle, Alkylidenbisphenole, Thiobisphenole, sekundäre aromatische Amine, Aminophenole, sterisch gehinderte Amine, Phosphite oder Phosphonite sind.

9. Orthopädische Klemmverbindung nach einem der vorhergehenden Ansprüche, **dadurch gekennzeichnet**, daß die Zwischenschicht (3, 4) zur Erhöhung der Benetzung der Oberfläche in der Rohrmuffe (1) ein Benetzungsmittel enthält.

10. Orthopädische Klemmverbindung nach einem der vorhergehenden Ansprüche, **dadurch gekennzeichnet**, daß die Zwischenschicht (3, 4) als Beschichtung fest auf der Rohrmuffe (1) oder auf dem Leichtmetallrohr (2) verankert ist.

11. Orthopädische Klemmverbindung nach einem der Ansprüche 1 bis 10, **dadurch gekennzeichnet**, daß die Zwischenschicht als separate Manschette ausgeführt ist.

12. Orthopädische Klemmverbindung nach einem der vorhergehenden Ansprüche, **dadurch gekennzeichnet**, daß der Hilfsstoff vom Aufbau mit dem Trägermaterial identisch ist.

13. Orthopädische Klemmverbindung nach einem der Ansprüche 1 bis 12, **dadurch gekennzeichnet**, daß der Hilfsstoff organisch aufgebaut ist.

14. Orthopädische Klemmverbindung nach Anspruch 13, **dadurch gekennzeichnet**, daß der organische Hilfsstoff Teer enthält.

15. Orthopädische Klemmverbindung nach Anspruch 13, **dadurch gekennzeichnet**, daß der organische Hilfsstoff aus duromeren Harzen aufgebaut ist.

16. Orthopädische Klemmverbindung nach Anspruch 15, **dadurch gekennzeichnet**, daß es sich bei den duromeren Harzen um Phenol-Formaldehyd (PF), Harnstoff-Formaldehyd (UF), Melamin-Formaldehyd (MF), ungesättigte Polyesterharze (UP), Epoxidharze (EP) oder vernetzte Polyurethane handelt.

17. Orthopädische Klemmverbindung nach Anspruch 13, **dadurch gekennzeichnet**, daß der organische Hilfsstoff aus thermoplastischen Kunststoffen aufgebaut ist.

18. Orthopädische Klemmverbindung nach Anspruch 17, **dadurch gekennzeichnet**, daß es sich bei den thermoplastischen Kunststoffen um Ethylen-Vinylacetat-Copolymer, Polyamid 11, Polyamid 12, Polyvinylidenfluorid, Tetrafluorethylen-Hexafluorpropylen-Copolymer Polychlortrifluorethylen handelt.

19. Orthopädische Klemmverbindung nach einem der vorhergehenden Ansprüche, **dadurch gekennzeichnet**, daß das Trägermaterial aus haftreibungserhöhenden Stoffen aufgebaut ist.

20. Orthopädische Klemmverbindung nach Anspruch 19, **dadurch gekennzeichnet**, daß die Haftstoffe eine höhere Härte aufweisen als das Leichtmetallrohr.

21. Orthopädische Klemmverbindung nach Anspruch 19 oder 20, **dadurch gekennzeichnet**, daß die haftreibungserhöhenden Stoffe anorganische Stoffe sind.

22. Orthopädische Klemmverbindung nach Anspruch 21, **dadurch gekennzeichnet**, daß die anorganischen Stoffe Mineral-, Glas-, Kohlenstoff- oder Metallfasern sowie Bariumsulfat oder Steinmehl sind.

23. Orthopädische Klemmverbindung nach einem der vorhergehenden Ansprüche, **dadurch gekennzeichnet**, daß die Zwischenschicht (3, 4) eine Schichtstärke von 0,1 µm bis 1 mm aufweist.

24. Orthopädische Klemmverbindung nach einem der vorhergehenden Ansprüche, **dadurch gekennzeichnet**, daß die Zwischenschicht (3, 4) als Isolationsschicht mit einem Durchgangswiderstand > 10¹⁰ Ω mm² ausgelegt ist.
